(19) Europäisches Patentamt
European Patent Office
Office européen des brevets

(11) Numéro de publication: **0 157 657**
**B1**

# (12) FASCICULE DE BREVET EUROPEEN

(45) Date de publication du fascicule du brevet: **14.09.88**

(21) Numéro de dépôt: **85400048.6**

(22) Date de dépôt: **11.01.85**

(51) Int. Cl.[4]: **C 07 F 15/06**, C 07 C 29/48, C 07 F 15/00, C 07 C 29/50, C 07 C 45/27, C 07 C 45/32

(54) **Complexes peroxydiques de métaux, leur préparation et leur utilisation dans les réactions d'oxydations d'hydrocarbures en alcools et/ou cetone.**

(30) Priorité: **31.01.84 FR 8401587**
**02.02.84 FR 8401736**

(43) Date de publication de la demande:
**09.10.85 Bulletin 85/41**

(45) Mention de la délivrance du brevet:
**14.09.88 Bulletin 88/37**

(84) Etats contractants désignés:
**DE GB NL**

(56) Documents cités:
**EP - A - 0 027 937**
**US - A - 3 816 548**

**INORGANIC CHEMISTRY, vol. 23, no. 1, 4 janvier 1984, pages 65-74, American Chemical Society; R.R. GAGNE et al.: "Ruthenium complexes of 1,3-bis(2-pyridylimino)isoindolines as alcohol oxidation catalysts"**
**INORGANIC CHEMISTRY, vol. 20, no. 10, octobre 1981, pages 3260-3267, American Chemical Society; R.G. GAGNE et al.: "Mononuclear and binuclear metal complexes of 1,3-bis(2-pyridylimino)isoindolines"**

(73) Titulaire: **INSTITUT FRANCAIS DU PETROLE, 4, Avenue de Bois-Préau, F-92502 Rueil-Malmaison (FR)**

(72) Inventeur: **Saussine, Lucien, 27, 29, avenue de Brimon, F-78400 Chatou (FR)**
Inventeur: **Robine, Alain, 10, rue Massena, F-92500 Rueil Malmaison (FR)**
Inventeur: **Mimoun, Hubert, 34, rue Hippolyte Bisson, F-92500 Rueil Malmaison (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 157 657 B1

ACTORUM AG

## Description

La présente invention concerne de nouveaux complexes peroxydiques d'un métal de formule générale

[I]LnMXpY

dans laquelle Y est un groupe peroxo, mu-peroxo ou un groupe de formule OOR, dans laquelle R représente un groupe alkyl ou aralkyl, L est un ligand défini ci-dessous, M est un métal, X un groupe anionique, avec $n=1$ ou 2, $p=0$ ou 1 et $n+p=2$, ainsi que l'utilisation de ces complexes comme réactifs d'oxydation ou comme catalyseurs d'oxydation des hydrocarbures par l'oxygène, par un peroxyde ou par un hydroperoxyde et en particulier un hydroperoxyde organique.

Les complexes peroxydiques peuvent catalyser notamment l'hydroxylation d'un hydrocarbure R'H par un hydroperoxyde R"OOH.

Les groupes R' et R" sont identiques ou différents du radical R et choisis parmi les groupes alkyl, cycloalkyl, aralkyl, alkenyl, cycloalkenyl et aralkenyl.

Dans les complexes précités, L est un ligand chélatant tridenté de formule générale (A) schématisée arbitrairement par BpI lorsque $R_1$ à $R_{12}$ représentent chacun un atome d'hydrogène.

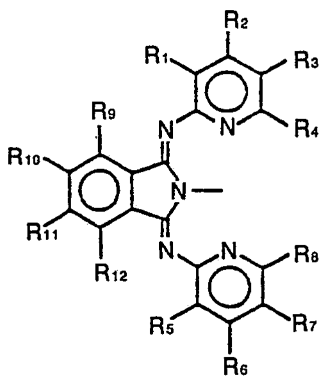

(A)

Dans cette formule (A) les symboles $R_1$ à $R_{12}$ représentent indépendamment les uns des autres un atome d'hydrogène, un groupe alkyl de $C_1$ à $C_{12}$, aryl de $C_6$ à $C_{14}$, aralkyl de $C_7$ à $C_{30}$, alkoxy de $C_1$ à $C_{12}$, alkoxyalkyl de $C_2$ à $C_{20}$, un atome d'halogène (Cl, F, Br, I) ou un groupe nitro ($-NO_2$).

Deux des groupes adjacents $R_1$ à $R_{12}$ pouvant ensemble former avec les atomes de carbone auxquels ils sont liés, un noyau aromatique. Lorsque $n=2$ les deux ligands L pouvant être identiques ou différents.

Les ligands L préférés sont ceux dans lesquels soit les groupes $R_1$ à $R_{12}$ représentent chacun un atome d'hydrogène, soit les groupes $R_2$ et $R_6$ identiques ou différents représentent chacun un groupe méthyle ou tertiobutyle, et sont de préférence identiques; le groupe $R_{11}$ représente un groupe méthyle ou tertiobutyle et les groupes $R_1$, $R_3$, $R_4$, $R_5$, $R_7$ à $R_{10}$ et $R_{12}$ représentent chacun un atome d'hydrogène.

X représente un groupe anionique choisi parmi:

– un anion carboxylate lui même choisi parmi l'acétate, le propionate, le benzoate, le pivalate, le stéarate, l'éthyl-2 hexanoate, l'octoate, le naphténate, le nitrobenzoate ou le trifluoracétate,

– un anion hydroxyde, mu-oxyde ou mu-peroxyde.

X est de préférence un groupe carboxylate.

Y est un groupe mu-peroxo, peroxo, alkylperoxo ou aralkylperoxo de formule ROO dans laquelle R est un groupe alkyl ou aralkyl.

Y est de préférence un groupe alkylperoxo ou aralkylperoxo, R est de préférence un groupe tertiaire tel que par exemple un groupe tertiobutyle ou cumyle (phényl-1 méthyl-1 éthyle).

M représente un métal choisi dans le groupe constitué par les métaux des groupes VIII, $I_B$ et $VII_B$ de la classification périodique des éléments (Handbook of Chemistry and Physics, 37e édition, 1955–56, pages 192–193).

M est avantageusement choisi parmi les métaux suivants: cobalt, fer, manganèse et cuivre et d'une façon préférée M représente le cobalt.

Parmi les ligands 1,3-bis (2'-pyridylimino)isoindolines dénommées ci-après (BpI) H utilisables dans la préparation des complexes selon l'invention, on peut par exemple utiliser les composés suivants:

1,3 bis (2'-pyridylimino)isoindoline: (BpI)H

1,3 bis (4'-méthyl-2'-pyridylimino)isoindoline: (4'Me-BpI)H

1,3 bis (4't-butyl-2'-pyridylimino)isoindoline: (4'tBu-BpI)H

1,3 bis (5'-chloro-2'-pyridylimino)isoindoline: (5'Cl-BpI)H

5-t-butyl-1,3 bis (2'-pyridylimino)isoindoline: (5 tBu-BpI)H

5-t-butyl-1,3 bis (4'-méthyl-2'-pyridylimino) isoindoline: (5 tBu-4'Me-BpI)H

5-t-butyl-1,3- bis (4'-butyl-2'-pyridylimino) isoindoline: (5 tBu-4'tB-BpI)H

Les complexes particulièrement préférés ont pour formules:

(BpI)Co(OAc)OOtBu
(BpI)Co(OBz)OOtBu
(BpI)Co(p-$NO_2$BzO)OOtBu
(4'Me-BpI)Co(OAc)OOtBu,
(4'Me-BpI)Co(OBz)OOtBu
(5'tBu-BpI)Co(OAc)OOtBu,
(5tBu-BpI)-Co(OBz)OOtBu
(5'Cl-BpI)Co(OAc)OOtBu
(BpI)Co(octoate)OOtBu
(BpI)Co(OAc)OO cumyl,
(BpI)Co(OBz)OO cumyl
(BpI)Fe(OAc)OOtBu, $(BpI)_2$Fe(OOtBu)
(BpI)Mn(OAc)OOtBu, (BpI)Mn(OBz)OOtBu
$(BpI)_2$MnOOtBu, $(4'Me\text{-}BpI)_2$MnOOtBu

dans lesquelles les symboles BpI, 4'Me-BpI, 5'Cl-BpI, 5tBu-BpI ont la signification ci-dessus et les symboles OAc, OBz, p-$NO_2$BzO représentent respectivement les groupes acétate, benzoate, et p-nitro-benzoate, Me et tBu représentent respectivement les groupes méthyle et tertiobutyle.

La préparation des complexes qui sera décrite de façon plus détaillée dans les exemples, peut s'effectuer de différentes façons.

Les ligands 1,3 bis (2'-pyridylimino) isoindoline peuvent être préparés par la méthode décrite par W.O. Siegl, J. Org. Chem. 42 (11), 1872 (1977).

Les complexes des métaux de transition dans l'état divalent LMX et $L_2M$ peuvent être obtenus selon les différentes préparations décrites par W.O. Siegl (référence ci-dessus) et plus récemment par R.R. Gagne et al., Inorg. Chem 20, 3260-7 (1981), avec certaines modifications selon la nature du groupement X ou des substituants sur le ligand L.

La synthèse en une seule étape des complexes LMX peut être réalisée par une réaction de la 2-aminopyridine sur le phtalonitrile en présence du sel de métal de transition $MX_2nH_2O$. Le produit est en général obtenu sous forme de précipité et une purification est nécessaire pour éliminer le complexe phtalocyanine, très peu soluble, qui se forme en faible quantité. Dans le cas des complexes LCo(OAc), on peut par exemple, dissoudre le précipité dans l'acide acétique, filtrer puis évaporer le filtrat obtenu sous pression réduite.

Les complexes alkylperoxo par exemple du cobalt (III) de formule générale LCoX(OOR) peuvent être facilement préparés par addition d'un hydroperoxyde à une suspension de complexe LCoX dans un solvant peu polaire tel que le dichlorométhane à une température de −40 à +80 °C environ et de préférence entre −15 et +40 °C; il y a solubilisation assez rapide accompagnée d'une intensification de la coloration rouge.

Les complexes LCoX(OOR) sont ensuite obtenus facilement par concentration de la solution.

Cette transformation peut être effectuée dans la plupart des solvants usuels qui ne réagissent pas avec les hydroperoxydes. On peut citer à titre d'exemples non limitatifs: le benzène, le chlorobenzène, le dichloro-1,2 éthane.

Ces complexes sont suffisamment stables pour être isolés purs et conservés au réfrigérateur pendant plusieurs semaines.

Un des avantages des complexes LnMXpY de l'invention est qu'ils sont en général beaucoup plus solubles que les complexes divalents LMX ou $L_2M$.

Une des propriétés les plus caractéristiques des complexes selon l'invention est qu'ils peuvent céder stoéchiométriquement de l'oxygène à un substrat hydrocarboné pouvant être une oléfine, un hydrocarbure saturé aliphatique ou un hydrocarbure aromatique.

Ces complexes ont la propriété d'oxyder stoéchiométriquement les alcanes linéaires ou ramifiés en un mélange d'alcools et de cétones. Dans le cas des alcanes ramifiés, on obtient principalement des alcools tertiaires.

Les oléfines sont principalement hydroxylées en position allylique. Les hydrocarbures alkyl ou cycloalkyl aromatiques sont oxydés en position benzylique essentiellement en un mélange d'alcool et de cétone.

Le rapport pondéral, substrat à oxyder sur complexe, est généralement compris entre 1:1 et 500:1, de préférence entre 10:1 et 250:1.

La température de réaction est comprise entre environ 0 °C et 180 °C, de préférence entre 20 et 100 °C.

La réaction est en général, effectuée sous azote, la pression totale dépend de la nature du substrat, du solvant et de la température choisie.

Une autre propriété caractéristique de ces complexes est qu'ils peuvent être régénérés à partir de leurs formes réduites correspondantes par oxydation à l'aide d'un oxydant et peuvent ainsi servir de catalyseur d'oxydation.

On peut ainsi oxyder catalytiquement:

– Un alcane linéaire ou ramifié, cyclique ou polycyclique, liquide ou gazeux contenant de préférence de deux à vingt atomes de carbone par exemple l'isobutane, l'isopentane, le butane, l'hexane, l'octane.

– Une oléfine linéaire ou cyclique contenant ou non une ramification ou une autre fonction comme par exemple une fonction cétone conjuguée ou non avec la double liaison, par exemple, le propène, les butènes, le cyclohexène, le méthyl-1-cyclohexène, l'alpha et le béta pinène.

– Un hydrocarbure alkyl ou cycloalkyl aromatique possédant ou non un ou plusieurs autres substituants sur le noyau aromatique, par exemple, l'éthylbenzène, le toluène et la tétraline.

Les catalyseurs selon l'invention sont particulièrement bien adaptés pour la transformation des isoparaffines en alcools tertiaires.

En particulier il est bien connu dans la littérature antérieure que l'isobutane peut être oxydé thermiquement en l'absence ou en présence de catalyseur pour préparer un mélange de tertiobutanol et d'hydroperoxyde contenant également de l'acétone, du méthanol et du peroxyde de ditertiobutyle tBuOOtBu, voir par exemple:

JK. Kochi et R.A. Sheldon ch. II – Metal catalyzed oxidations of organic compounds, Academic Press (1981).

D.E. Winkler et al., US 2 845 461 (1958), ont décrit des conditions permettant d'obtenir un bon rapport hydroperoxyde sur tertiobutanol.

BJ. Barone et al., US 3 974 228 (1976), utilisent l'oxyde de Lanthane pour augmenter la sélectivité en hydroperoxyde lors de l'oxydation des alcanes tertaires.

A.M. Brownstein et al., US 4 028 423 (1977), obtiennent une bonne sélectivité en hydroperoxyde en catalysant l'oxydation de l'isobutane par une polyphtalocyanine de cuivre activée par une amine aromatique.

Les brevets récents de EG. Foster et al., EP-76 532, EP-76533 et EP 76534 (1983), décrivent une amélioration de la productivité en hydroperoxyde de tertiobutyle, tout en conservant une bonne sélectivité pour des faibles conversions de l'isobutane, en opérant au-dessus de la température et de la pression critiques.

Il est connu que l'oxydation de l'isobutane en présence de sels solubles de cobalt (octoate ou naphténate de cobalt [II]) est plus rapide que l'oxydation sans catalyseur et conduit principalement au tertiobutanol.

D.E. Winkler et G.W. Hearne (Ind. Eng. Chem. 53 [1961], 655-8) ont montré que dans ce cas, il y a formation accrue de produits de coupure (acétone, méthanol, acides). La diminution du rendement total en hydroperoxyde de tertiobutyle et tertiobutanol est de l'ordre de 10% per rapport à l'oxydation sans catalyseur.

B.E. Johnston et al., US 3 825 605 (1974), utilisent un catalyseur solide qui est un oxyde de molybdène ou de tungstène contenant d'autres métaux (Fe, Co, Cr, Al) en faible proportion.

R.H. Williams et al., US 3 816 548 (1974), utilisent les phtalocyanines de divers métaux (Mn, Fe, Co, Cu) pour oxyder les isoparaffines principalement en alcools tertiaires.

Les phtalocyanines de différents métaux de transition sont bien connues pour décomposer les hydroperoxydes intermédiaires obtenus lors de l'oxydation d'hydrocarbures tels que le cyclohexène, l'éthylbenzène, le cumène.

Des complexes de métaux de transition ayant une structure apparentée aux phtalocyanines, ont été revendiqués comme catalyseurs pour la décomposition de l'hydroperoxyde de cyclohexyle en cyclohexanol et cyclohexanone en présence de cyclohexane; J.D. Druliner et al., US 4 326 084 (1982), correspondant à la demande européenne EP 27937 (1981).

H.R. Grane et al., US 4 294 999 (1981), et US 4 296 262 (1981), oxydent l'isobutane en présence d'un sel soluble de molybdène pour obtenir un mélange contenant environ 3 fois plus de tertiobutanol que d'hydroperoxyde. Ils décomposent l'hydroperoxyde par traitement thermique et obtiennent ainsi un effluent qui contient 80% de tertiobutanol, 10% d'acétone et 5,5% de méthanol en poids par rapport au poids total de l'effluent.

Ces auteurs ont également revendiqué US 4 296 263 (1981) une extension de cette méthode à l'oxydation de mélanges d'isobutane et de n-butane en présence de sels solubles de divers métaux (Cr, Co, Ni, Mn) les conditions utilisées permettant de minimiser l'oxydation du n-butane. L'acétone, le méthanol et d'autres sous produits oxygénés codistillent avec le tertiobutanol et se retrouvent dans le produit final.

R.T. Adams et al., US 3 829 510 (1974), oxydent l'isobutane dans l'acide acétique en présence d'un catalyseur choisi parmi les sels de divers métaux (Co, Cu, Mn...) avec recyclage de l'acétate de méthyle et hydrogénation du mélange tertiobutanol, acétone, méthanol, acétate de méthyle pour transformer l'acétone en alcool isopropylique et obtenir un mélange utilisable dans les carburants.

L'utilisation de catalyseurs pour l'oxydation d'isoparaffines conduit à des oxydats riches en alcools tertiaires mais non exempts d'hydroperoxydes et de sous produits. Pour la production directe de tertiobutanol, il est important d'obtenir un rapport tertiobutanol sur hydroperoxyde de tertiobutyle maximum, tout en conservant un bon rendement. D'autant plus, que la décomposition thermique de l'hydroperoxyde de tertiobutyle qui a été étudiée par N.A. Milas et D.M. Surgenor (J. Am. Chem. Soc. 68, 205 [1946]) ne fournit que 85% de tertiobutanol alors que celle du peroxyde de ditertiobutyle donne principalement de l'acétone.

L'utilisation des nouveaux complexes, en tant que catalyseurs selon la présente invention permet d'obtenir par oxydation de l'isobutane par l'oxygène de l'air, en présence de l'un des complexes définis ci-dessus, un mélange de tertiobutanol et d'hydroperoxyde de tertiobutyle beaucoup plus riche en tertiobutanol que les mélanges obtenus selon les techniques antérieures.

L'utilisation des complexes selon l'invention permet également d'effectuer la réaction à des températures moins élevées que celles de la technique antérieure tout en conservant une vitesse de réaction élevée, cela entraînant une diminution de la formation de sous-produits. Le catalyseur employé dans le procédé selon l'invention a de plus une durée de vie supérieure à celle des sels solubles de cobalt, par exemple l'octoate employé dans l'art antérieur.

Les catalyseurs selon la présente invention peuvent intervenir soit directement dans l'étape d'oxydation, soit dans la décomposition de l'hydroperoxyde ROOH en présence de l'isoparaffine de façon à obtenir une plus grande quantité d'alcool.

A. L'oxydant est un hydroperoxyde.

La réaction peut être effectuée en l'absence ou en présence d'un solvant comme par exemple, le benzène, le 1,1,2-trichloro-2,2,1-trifluoroéthane.

La quantité de catalyseur est comprise entre 1 et $10^4$ ppm de complexe par rappport au substrat à oxyder et de préférence entre 1 et 5000 ppm et plus particulièrement entre 10 et 500 ppm.

La température de réaction est comprise entre −20 °C et +180 °C et de préférence entre 0 et 100 °C.

L'hydroperoxyde est ajouté lentement de sorte que sa concentration dans le milieu réactionnel reste faible, c'est-à-dire de 0,1 à 10% environ en poids par rapport au poids du mélange réactionnel et de préférence de 0,5 à 5%. Il peut être ajouté pur ou en solution dans le composé à oxyder ou dans un autre solvant.

B. L'oxydant est l'oxygène.

Dans ce cas on se place dans les conditions classiques où l'hydrocarbure R'H est oxydé en hydroperoxyde R'OOH, le catalyseur $L_nMX_pY$ intervient pour décomposer l'hydroperoxyde en alcool tout en hydroxylant l'hydroarbure R'H comme cela est montré par les essais stoéchiométriques dans les exemples.

La température de réaction est comprise entre environ 40 °C et environ 180 °C, et de préférence entre 80 °C et 160 °C environ. La réaction est de préférence en général initiée par un initiateur de radicaux libres, et d'une façon avantageuse par un peroxyde ou un hydroperoxyde à la concentration de 0,1 à 1% en poids par rapport à l'hydrocarbure; d'une manière particulièrement avantageuse le peroxyde ou l'hydroperoxyde employé est celui dont la partie hydrocarbonée a la même structure que l'hydrocarbure à oxyder.

Dans le cas de l'oxydation de l'isobutane, la réaction s'effectue en phase liquide sous une pression comprise généralement entre 1 et 10 mégapascals (MPa) environ et de préférence entre 2 et 6,5 MPa.

Dans le cas de l'isobutane, la réaction est de préférence initiée par le peroxyde de ditertiobutyle ou l'hydroperoxyde de tertiobutyle (0,1 à 1% en poids par rapport à l'isobutane).

La pression partielle d'oxygène est en général comprise entre environ 0,05 et 0,5 MPa et de préférence entre 0,15 et 0,4 MPa. L'oxygène est utilisé

pur ou dilué avec un gaz inerte, par exemple l'azote, ou avec un mélange de gaz inerte. On peut également employer l'air ou l'air enrichi par de l'oxygène ou l'air dilué par un gaz inerte par exemple de l'azote. On préfère généralement utiliser l'air seul.

La quantité pondérale de catalyseur est comprise entre environ 1 et 5000 ppm de complexe par rapport à l'hydrocarbure à oxyder, plus particulièrement entre 10 et 500 ppm.

Le catalyseur peut être introduit en totalité au début de la réaction ou par petites portions pendant toute sa durée.

La réaction peut être réalisée en réacteur de type continu ou discontinu.

La présente invention est illustrée par les exemples suivants:

Exemple 1: Préparation du complexe numéro 1 (Bpl)Co(OAc)OOtBu

2 ml d'hydroperoxyde de tertiobutyle (tBuOOH) contenant 20% de peroxyde de ditertiobutyle (tBuOOtBu) sont ajoutés goutte à goutte à 20 °C à 1 g de complexe (Bpl)Co(OAc) (2,4 mmoles) placé en suspension dans le dichlorométhane.

Après une heure de réaction à 20 °C, la solution rouge obtenue est séchée sur $Na_2SO_4$. Après filtration et concentration sous vide le produit est précipité par addition de 10 ml d'éther anhydre. Il est filtré puis lavé 4 fois par un mélange éther-pentane (⅓) à 0 °C et séché sous vide sur $P_2O_5$. On recueille 1 g de complexe (rendement molaire par rapport au complexe de départ: 82,5%).

Analyse infrarouge:
(O–O) 880 $cm^{-1}$, (C–H) = 2980 $cm^{-1}$

Analyse de résonance magnétique nucléaire dans le dichlorométhane deutérié.
(RMN) delta (ppm): 0,47(s,9H); 1,64(s,3H) 7,1 à 8,2 (m,10H); 8,9 (d,2H).
Analyse élémentaire: $C_{24}H_{24}N_5O_4Co$
Calculée pour la formule:

| | | | | |
|---|---|---|---|---|
| | C 57,04 | H 4,75 | N 13,86 | O 12,67 |
| Trouvée: | C 57,14 | H 4,90 | N 13,77 | O 12,60 |

Exemple 2: Préparation du complexe numéro 2 (Bpl)Co(OAc)OO cumyl

La synthèse est effectuée suivant le mode opératoire de l'exemple 1. Addition à 0 °C (10 minutes) de 4,2 ml d'hydroperoxyde de cumyle à 80% à une suspension de 2 g du complexe (Bpl) Co OAc (4,8 mmoles) dans 50 ml de benzène. Après 1 h 30 à 20 °C, on sépare par filtration 0,6 g de complexe (Bpl)Co OAc (30%). La solution rouge conduit après un traitement identique à celui de l'exemple 1 à 1,65 g de cristaux marrons (60,5%).

Analyse infrarouge: (O–O) = 840 $cm^{-1}$
Analyse RMN: delta (ppm): 0,76(s,6H); 1,64(s,3H) 6,6–8,2 (m,15H); 8,9 (d,2H).
Analyse élémentaire: $C_{29}H_{26}N_5O_4Co$
Calculée pour la formule:

| | | | | |
|---|---|---|---|---|
| | C 61,38 | H 4,58 | N 12,35 | O 11,29 |
| Trouvée: | C 61,02 | H 4,70 | N 11,99 | O 11,24 |

Exemple 3: Préparation du complexe numéro 3 (Bpl)Co(OBz)OOtBu

7,5 g (15,7 mmoles) de complexe (Bpl)Co OBz sont placés en suspension dans le dichlorométhane (150 ml) et traités à 20 °C par 10 ml d'hydroperoxyde de tertiobutyle à 80%. Après 4 h d'agitation à 20 °C il y a pratiquement solubilisation totale, on sépare par filtration 0,4 g (5,3%) de complexe de départ. Par un traitement de la solution identique à celui de l'exemple 1, on obtient 8,3 g de solide marron (93,3%).

Analyse infrarouge: (O–O) 870 $cm^{-1}$, (C–H) 2980 $cm^{-1}$
Analyse RMN: delta (ppm): 0,52 (s,9H); 7–8,2 (m, 15H); 9(d,2H)
Analyse élémentaire: $C_{29}H_{26}N_5O_4Co$
Calculée pour la formule:

| | | | | |
|---|---|---|---|---|
| | C 61,38 | H 4,58 | N 12,35 | O 11,29 |
| Trouvée: | C 60,35 | H 4,51 | N 12,01 | O 11,2 |

Exemple 4: Préparation du complexe numéro 4 (Bpl)Co(OBz)OO Cumyl

5 g (10,5 mmoles) de complexe (Bpl)CoOBz sont traités à 20 °C par 6,5 ml d'hydroperoxyde de cumyle à 80% dans 90 ml de dichlorométhane pendant 7 heures jusqu'à dissolution totale. Après traitement le produit est obtenu par cristallisation à 0 °C dans un mélange éther-pentane (1/1) après séparation d'une impureté moins soluble (1,1 g). On obtient 3 g de produit (45,5%).

Analyse infrarouge: (O–O) 880 $cm^{-1}$
Analyse RMN: delta (ppm): 0,68(s,6H); 6,6–8,2 (m,15H) 9,05(d,2H)
Analyse élémentaire: $C_{34}H_{28}N_5O_4Co$
Calculée pour la formule:

| | | | | |
|---|---|---|---|---|
| | C 64,87 | H 4,45 | N 11,13 | O 10,17 |
| Trouvée: | C 63,85 | H 4,28 | N 11,02 | O 10,07 |

Exemple 5: Préparation du complexe numéro 5 (Bpl)Co(p-$NO_2$BzO) OOtBu

400 mg (0,76 mmoles) de complexe (Bpl)Co(p-$NO_2$BzO) en suspension dans 10 ml de dichlorométhane sont traités à 20 °C par 0,5 ml de tBuOOH à 80% pendant 2h.

La solution rouge obtenue est traitée comme dans l'exemple no 1 et conduit à 300 mg de complexe (64%).

Analyse infrarouge: (O–O) 850 $cm^{-1}$, (C–H) 2980 $cm^{-1}$
Analyse RMN: delta (ppm): 0,57(s,9H); 7–8,6 (m,14H) 8,9(d,2H)
Analyse élémentaire: $C_{29}H_{25}N_6O_6Co$
Calculée pour la formule:

| | | | | |
|---|---|---|---|---|
| | C 56,87 | H 4,08 | N 13,72 | O 15,69 |
| Trouvée: | C 56,14 | H 4,15 | N 13,55 | O 15,39 |

Exemple 6: Préparation du complexe no 6 (4'Me-Bpl)Co(OAC)OOtBu.

Ce complexe est préparé à 0 °C à partir de 3,55 g (8 mmoles) de complexe (4'Me-Bpl)CoOAC en suspension dans 70 ml de dichlorométhane et 6 ml de tBuOOH à 80%. Il y a solubilisation totale après 15 minutes. Au bout de 45 minutes à 0 °C, un traitement comme dans l'exemple no 1 conduit à 4 g de précipité marron (94%).

Analyse infrarouge: (O–O) 878 $cm^{-1}$, (C–H) 2970 $cm^{-1}$

Analyse RMN: delta (ppm): 0,5(s,9H); 1,64 (s,3H); 2,58(s,6H); 6,9 à 8,2 (m,8H); 8,7 (d,2H).
Analyse élémentaire: $C_{26}H_{28}N_5O_4Co$
Calculée pour la formule:

| | | | | |
|---|---|---|---|---|
| | C 58,54 | H 5,25 | N 13,13 | O 12,01 |
| Trouvée: | C 58,27 | H 5,36 | N 13,11 | O 12,6 |

Exemple 7: Préparation du complexe no 7 (4'Me-Bpl)Co(OBz)OOtBu

Ce complexe est préparé à 0 °C à partir de 2,1 g (4,15 mmoles) de complexe (4'Me-Bpl)CoOBz dans 40 ml de dichlorométhane et 3 ml de tBuOOH à 80%. Après 3 h à 0 °C la solubilisation est presque totale et le produit de départ non dissous 150 mg (7,1%) est séparé par filtration. Le traitement de la solution suivant le mode opératoire défini à l'exemple 1, conduit à 2,2 g de précipité marron (89%).
Analyse infrarouge: (O–O) 863 $cm^{-1}$, (C–H) 2975
Analyse RMN: delta (ppm): 0,55(s,9H), 2,55 (s,6H) 6,9 à 8,4 (m,13H) 8,86 (d,2H).
Analyse élémentaire: $C_{31}H_{30}N_5O_4Co$
Calculée par la formule:

| | | | | |
|---|---|---|---|---|
| | C 62,53 | H 5,04 | N 11,76 | O 10,75 |
| Trouvée: | C 62,14 | H 5,10 | N 11,32 | O 11,07 |

Exemples 8 à 19: Oxydations stoéchiométriques des alcanes par les complexes peroxydiques.

Dans un réacteur calorifugé, on introduit une millimole de complexe dans 25 ml de substrat à oxyder. Le mélange est dégazé et chauffé sous atmosphère d'azote.

Les produits sont dosés par chromatographie en phase vapeur (CVP) avec étalonnage interne après destruction éventuelle du complexe non transformé par la triphényl phosphine en excès.

Les résultats sont rassemblés dans le tableau I. Les rendements sont exprimés par rapport au complexe.

Exemples 20 à 26: Hydroxylation des alcanes par les hydroperoxydes catalysée par les complexes peroxydiques.

Dix millimoles d'hydroperoxyde, pur ou en solution, sont ajoutées peu à peu sous azote pendant ½ heure à une solution de 0,01 millimole de catalyseur dans 25 ml d'alcane à oxyder à 80 °C. Le mélange est agité encore 1 heure à 80 °C et analysé par CPV.

Les résultats sont rassemblés dans le tableau II. On note une amélioration très nette du rendement en produit d'hydroxylation par rapport à l'essai témoin (exemple 25) pour une même concentration en cobalt.

Les rendements sont exprimés par rapport à l'hydroperoxyde.

Exemple 27:

On ajoute peu à peu pendant 30 minutes, 0,1 mole d'hydroperoxyde de tertiobutyle dilué dans 10 ml de 1,1,2-trichlorotrifluoroéthane, à 65 g d'isobutane, contenant 100 ppm de complexe (Bpl)Co(OBz)OOtBu, maintenu à 90 °C. Après un temps de réaction total de 90 minutes, le mélange est refroidi et analysé.

Le dosage iodométrique montre la disparition presque totale de l'hydroperoxyde (95%). Le dosage CPV montre la formation de 130% de tertiobutanol par rapport à l'hydroperoxyde chargé.

Exemples 28 à 41

80 g d'isobutane (1,38 mole) sont introduits dans un réacteur émaillé.

Le système est chauffé à la température de réaction indiquée dans le tableau 3. Quand la pression est stabilisée, on introduit 0,5 g de peroxyde de ditertiobutyle (0,6% en poids par rapport à l'isobutane mis en jeu) comme initiateur, et le catalyseur en solution dans 1 ml de tertiobutanol.

La pression partielle d'oxygène est fixée à 0,4 MPa.

La température et la pression sont maintenues constantes par un dispositif de régulation thermique et apport d'oxygène.

A la fin de l'essai, le mélange est refroidi à la température ambiante et dégazé. L'hydroperoxyde est dosé par iodométrie; le tertiobutanol, l'acétone et le méthanol sont dosés par chromatographie en phase vapeur (CPV) après réduction d'une prise d'essai par le triphényl phosphine en excès. Le tertiobutanol présent avant réduction est égal à la différence entre le tertiobutanol total après réduction et l'hydroperoxyde de tertiobutyle.

Dans les exemples 29 et 30, la totalité du catalyseur est injectée au début de la réaction. Pour l'exemple 31, le complexe est injecté en deux fois en solution dans le tertiobutanol au début et au milieu du temps de réaction.

Pour les essais 32 à 41, le catalyseur est injecté en solution dans le tertiobutanol en plusieurs fois (toutes les 30 mn pour les essais 32 à 35 et toutes les 45 minutes pour les essais 36 à 41). L'exemple 28 est un essai témoin, l'oxydation a lieu en l'absence de catalyseur. L'exemple 33 est un essai témoin en présence d'un catalyseur qui est l'octoate de cobalt. On note une augmentation de la vitesse et de la sélectivité en tertiobutanol par rapport aux deux essais témoins.

Les résultats sont rassemblés dans le tableau III.

La sélectivité (S) est exprimée en moles de chaque produit formé par rapport au nombre de moles d'isobutane converties.

La sélectivité S s'exprime donc par:

$$S = 100 \times \frac{\text{Nombre de moles de produit formé}}{\text{Nombre de moles d'isobutane converties}}$$

Tableau I

| Exemples | Complexes | Substrat | T°C | Durée min | Produit(s) et rendement(s) molaire par rapport au complexe en % |
|---|---|---|---|---|---|
| 8 | (Bpl)Co(OAc)OOtBu | Cyclohexane | 80 | 75 | cyclohexanol (30) cyclohexanone (16) |
| 9 | (Bpl)Co(OAc)OOcumyl | Cyclohexane | 80 | 75 | cyclohexanol (24) cyclohexanone (35) |
| 10 | (Bpl)Co(OBz)OOtBu | Cyclohexane | 80 | 75 | cyclohexanol (10) cyclohexanone (24) |
| 11 | (4Me-Bpl)Co(OAc)OOtBu | Cyclohexane | 80 | 75 | cyclohexanol (30) cyclohexanone (22) |
| 12 | (Bpl)Co(OBz)OOtBu | décaline-cis | 80 | 75 | décalol-9 cis(17) décalol-9 trans (60) |
| 13 | (Bpl)Co(OAc)OOtBu | octane | 80 | 75 | octanol[a] (18,5) octanone[b] (38,0) |
| 14 | (Bpl)Co(OAc)OOcumyl | isobutane | 80 | 60 | tertiobutanol (10) |
| 15 | (Bpl)Co(OAc)OOtBu | Cyclohexène | 40 | 75 | cyclohexène-2 ol-1(31)cyclohexène-2 one-1 (18) |
| 16 | (Bpl)Co(OBz)OOtBu | Cyclohexène | 40 | 120 | cyclohexène-2 ol-1(5) cyclohexène-2 one-1 (42) |
| 17 | (Bpl)Co(OAc)OOtBu | Ethylbenzène | 45 | 90 | phényl-1 éthanol (3,5) acétophénone (40) |
| 18 | (Bpl)Co(OBz)OOtBu | tétraline | 80 | 90 | alpha-tétralol (41) alpha-tétralone (33) |
| 19 | (4′Me-Bpl)Co(OBz)OOtBu | cyclohexane | 80 | 75 | cyclohexanol (15) cyclohexanone (35) |

[a] L'octanol est un mélange d'octanol 1,2,3 et 4.
[b] L'octanone est un mélange d'octanone 2,3 et 4.

Tableau II

| Exemples | Catalyseur | ppm/substrat Co. | Complexe | Hydroperoxyde | alcane | produit(s) (rendement molaire %) | Rend. total en %[a] |
|---|---|---|---|---|---|---|---|
| 20 | (Bpl)Co(OAc)OOtBu | 15 | 128 | t-butyl[b] | cyclohexane | cyclohexanol (28,5) cyclohexanone (16,5) | 61,5 |
| 21 | (4′Me-Bpl)Co(OAc)OOtBu | 15 | 135 | t-butyl[b] | cyclohexane | cyclohexanol (33) cyclohexanone (17) | 67 |
| 22 | (Bpl)Co(OBz)OOtBu | 15 | 144 | t-butyl[b] | cyclohexane | cyclohexanol (29) cyclohexanone (15) | 59 |
| 23 | (Bpl)Co(OAc)OOCumyl | 15 | 144 | cumyl[c] | cyclohexane | cyclohexanol (39) cyclohexanone (17) | 73 |
| 24 | (Bpl)Co(OAc)OOtBu | 15 | 128 | t-butyl[d] | cyclohexane | cyclohexanol (31) cyclohexanone (22) | 75 |
| 25 | $Co(oct)_2$ | 15 | | t-butyl[b] | cyclohexane | cyclohexanol (11,5) cyclohexanone (8,5) | 28,5 |
| 26 | (Bpl)Co(OAc)OOtBu | 15 | 128 | t-butyl[b] | octane | octanol (8,9) octanone (9,9)[e] | 28,7 |

[a] Le rendement total est calculé en considérant que la formation d'une mole de cétone nécessite 2 moles d'hydroperoxyde.
[b] On utilise l'hydroperoxyde de tertiobutyle pur à 98%.
[c] On utilise l'hydroperoxyde de cumyle à 80% dans le cumène.
[d] L'hydroperoxyde est introduit en solution dans le tertiobutanol (50% en volume).
[e] L'octanol est constitué d'un mélange d'octanol 1,2,3 et 4 et l'octanone d'un mélange d'octanone 2,3 et 4.

Tableau III

| Ex. | Catalyseur | ppm/isobutane métal | ppm/isobutane complexe | T°C | durée min. | convers. % | Selectivité (S) % tertio-butanol | Selectivité (S) % hydroperoxyde de tert-butyl | Selectivité (S) % acétone | Selectivité (S) % méthanol | tertio-butanol hydroperoxyde de tert-butyl |
|---|---|---|---|---|---|---|---|---|---|---|---|
| 28 | | 0 | 0 | 125 | 300 | 10,5 | 32,2 | 59,5 | 5,4 | 1,5 | 0,54 |
| 29 | (Bpl)Co(OBz)OOtBu | 10,4 | 100 | 125 | 23 | 5,3 | 73,4 | 9,2 | 5,3 | 5,8 | 7,97 |
| 30 | (Bpl)Co(OBz)OOtBu | 10,4 | 100 | 100 | 110 | 5,5 | 79,1 | 9,8 | 2,4 | 2,5 | 8,07 |
| 31 | (Bpl)Co(OBz)OOtBu | 20,8 | 200 | 110 | 310 | 16,5 | 85,6 | 9,3 | 6,4 | 3,9 | 9,2 |
| 32 | (Bpl)Co(OBz)OOtBu | 8,3 | 80 | 120 | 120 | 12,8 | 74,8 | 10,1 | 6,1 | 4,8 | 7,4 |
| 33 | $Co(oct)_2$ | 8,3 | – | 120 | 120 | 7,2 | 64,5 | 25,5 | 2,6 | 2,3 | 2,53 |
| 34 | (Bpl)Co(octoate) OOtBu | 8,3 | 83 | 120 | 120 | 13,4 | 74 | 10,3 | 6,4 | 5 | 7,18 |
| 35 | (Bpl)Co(OAc)OOtBu | 8,3 | 71 | 120 | 120 | 11,5 | 76,5 | 10,2 | 5,3 | 4,2 | 7,5 |
| 36 | (Bpl)Co(OAc)OOtBu | 8,3 | 71 | 110 | 180 | 12,2 | 82 | 9,2 | 6,1 | 3,9 | 8,9 |
| 37 | (Bpl)Co(OBz)OOtBu | 8,3 | 80 | 110 | 180 | 12,7 | 83,5 | 8,2 | 6,4 | 4,2 | 10,2 |
| 38 | (4'Me-Bpl)Co(OAc) OOtBu | 8,3 | 75 | 110 | 180 | 11,8 | 81,8 | 8,6 | 5,3 | 3,9 | 9,5 |
| 39 | (Bpl)Co(OAc)OOtBu | 8,3 | 71 | 110 | 180 | 12 | 83 | 10,5 | 5,5 | 3,9 | 7,9 |
| 40 | (Bpl)Co(OBz)OOtBu | 16,6 | 160 | 110 | 420 | 24 | 82,5 | 7,3 | 7,4 | 3,9 | 11,2 |
| 41 | (Bpl)Co(OBz)OOtBu | 10 | 96 | 120 | 240 | 21 | 80 | 8,8 | 8,2 | 5 | 9,1 |

**Revendications**

1. Complexes peroxydiques des métaux de formule générale LnMXpY I dans laquelle L représente un ligand de formule (A),

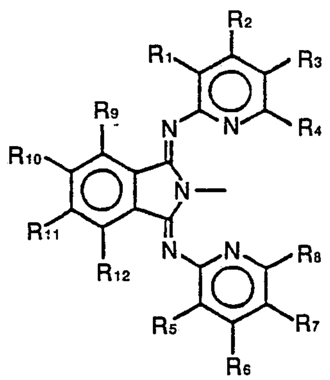

(A)

n = 1 ou 2, p = 0 ou 1 et n + p = 2, les deux ligands L pouvant être identiques ou différents lorsque n = 2.

$R_1$ à $R_{12}$ identiques ou différents sont choisis chacun dans le groupe constitué par un atome d'hydrogène, un groupe alkyl de $C_1$ à $C_{12}$, un groupe aryl de $C_6$ à $C_{14}$, un groupe aralkyl de $C_7$ à $C_{30}$, un groupe alkoxy de $C_1$ à $C_{12}$, un groupe alkoxy-alkyl de $C_2$ à $C_{20}$, un atome d'halogène et un groupe nitro.

Deux groupes $R_1$ à $R_{12}$ adjacent pouvant ensemble former avec les atomes de carbone auxquels ils sont liés, un noyau aromatique.

X représente un groupe anionique choisi parmi un anion hydroxyde, mu-oxyde ou mu-peroxyde ou un anion carboxylate lui-même choisi parmi l'acétate, le propionate, le benzoate, le pivalate, le stéarate, l'éthyl-2-hexanoate, l'octoate, le naphténate, le nitrobenzoate ou le trifluoroacétate.

M représente un métal choisi dans le groupe constitué par les métaux des groupes VIII, $I_B$ et $VII_B$.

Y est un groupe peroxo, mu-peroxo ou un groupe d eformule OOR dans laquelle R représente un groupe alkyl, ou aralkyl.

2. Complexes selon la revendication 1 dans lesquels M est un métal du groupe VIII, Y est un groupe de formule OOR dans laquelle R est un groupe alkyl ou aralkyl tertiaire.

3. Complexes selon la revendication 1 ou 2 dans lesquels dans le ligand L, $R_1$ à $R_{12}$ représentent chacun un atome d'hydrogène.

4. Complexes selon l'une des revendications 1 à 3 dans lesquels $R_2$ et $R_6$ représentent simultanément un groupe méthyle ou tertiobutyle, $R_{11}$ représente un groupe méthyle ou tertiobutyle, $R_1$, $R_3$, $R_4$, $R_5$, $R_7$ à $R_{10}$ et $R_{12}$ représentent chacun un atome d'hydrogène.

5. Complexes selon l'une des revendications 1 à 4 dans lesquels X est un groupe carboxylate, M est le cobalt et n = p = 1.

6. Utilisation d'au moins un complexe défini dans l'une des revendications 1 à 5 à titre de catalyseur, pour l'oxydation en alcools, en cétones ou en un mélange d'alcools et de cétones, d'oléfines, d'alcanes linéaires ou ramifiés, d'hydrocarbures alkyl ou cycloalkyl-aromatiques, par les hydroperoxydes organiques en présence ou en absence d'oxygène.

7. Utilisation d'au moins un complexe défini dans l'une des revendications 1 à 5 à titre de catalyseur pour l'oxydation en phase liquide d'isoparaffines en oxydats riches en alcools tertiaires, à l'aide d'un gaz contenant de l'oxygène.

8. Utilisation selon la revendication 7 dans une réaction d'oxydation par l'air, de l'isobutane en tertiobutanol.

9. Utilisation selon l'une des revendications 7 ou 8 dans laquelle la température est comprise entre 40 et 180 °C environ et la pression entre 1 et 10 MPa environ.

10. Utilisation selon l'une des revendications 7 à 9

dans laquelle on emploie de 0,1 à 1% en poids, par rapport à l'isoparaffine mise en jeu, d'hydroperoxyde ou de peroxyde de tertioalkyle correspondant, comme initiateur de la réaction.

11. Utilisation selon l'une des revendications 6 à 10, dans laquelle on emploie une quantité catalytique du complexe de 1 à 5000 ppm en poids par rapport au substrat à oxyder.

12. Utilisation à titre de réactif d'au moins un complexe défini dans l'une des revendications 1 à 5, pour l'oxydation en alcool, en cétone ou en un mélange d'alcools et de cétones, des alcanes linéaires ou ramifiés, des oléfines linéaires ou cycliques, des hydrocarbures alkyl ou cycloalkylaromatiques.

## Patentansprüche

1. Peroxydische Metallkomplexe der allgemeinen Formel LnMXpY [I], worin L einen Liganden der Formel (A) bedeutet,

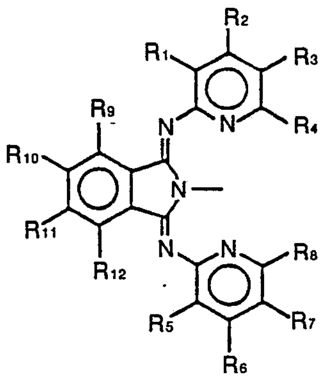

(A)

n = 1 oder 2, p = 0 oder 1 und n + p = 2 sind, wobei die beiden Liganden L gleich oder verschieden sein können, wenn n = 2 ist, $R_1$ bis $R_{12}$, die gleich oder verschieden sein können, jeweils aus der Gruppe Wasserstoffatome, $C_1$- bis $C_{12}$-Alkylgruppen, $C_6$- bis $C_{14}$-Arylgruppen, $C_7$- bis $C_{30}$-Aralkylgruppen, $C_1$- bis $C_{12}$-Alkoxygruppen, $C_2$- bis $C_{20}$-Alkoxyalkylgruppen, Halogenatome und Nitrogruppen gewählt sind, wobei zwei benachbarte Gruppen $R_1$ bis $R_{12}$ zusammen mit den Kohlenstoffatomen, an die sie gebunden sind, einen aromatischen Kern bilden können,

X eine anionische Gruppe bedeutet, die gewählt ist aus den Carboxylatanionen Acetat, Propionat oder Benzoat, Pivalat, Stearat, 2-Ethylhexanoat, Octoat, Naphthenat, Nitrobenzoat und Trifluoracetat oder einem der Hydroxid-, Mu-Oxid-, oder Mu-Peroxid-Anionen,

M ein Metall aus der Gruppe der Metalle der Gruppen VIII, $I_B$ und $VII_B$ bedeutet und

Y eine Peroxo-, Mu-Peroxo oder eine Gruppe der Formel OOR, worin R eine Alkyl- oder Aralkylgruppe bedeutet, darstellt.

2. Komplexe nach Anspruch 1, dadurch gekennzeichnet, daß M ein Metall der Gruppe VIII ist und Y eine Gruppe der Formel OOR darstellt, worin R eine tertiäre Alkyl oder Aralkylgruppe bedeutet.

3. Komplexe nach Anspruch 1 oder 2, dadurch gekennzeichnet, daß im Liganden L die Gruppen $R_1$ bis $R_{12}$ jeweils ein Wasserstoffatom bedeuten.

4. Komplexe nach einem der Ansprüche 1 bis 3, dadurch gekennzeichnet, daß $R_2$ und $R_6$ gleichzeitig eine Methylgruppe oder tert.Butylgruppe bedeuten, $R_{11}$ eine Methylgruppe oder tert.Butylgruppe darstellt und $R_1$, $R_3$, $R_4$, $R_5$, $R_7$ bis $R_{10}$ und $R_{12}$ jeweils ein Wasserstoffatom bedeuten.

5. Komplexe nach einem der Ansprüche 1 bis 4, dadurch gekennzeichnet, daß X eine Carboxylatgruppe ist, M Kobalt bedeutet und n = p = 1 ist.

6. Verwendung zumindest eines Komplexes nach einem der Ansprüche 1 bis 5 als Katalysator zur Oxidation von Olefinen, linearen oder verzweigten Alkanen oder von alkyl- oder cycloalkylaromatischen Kohlenwasserstoffen zu Alkoholen, Ketonen oder einem Gemisch von Alkoholen und Ketonen durch organische Hydroperoxide in Gegenwart oder bei Abwesenheit von Sauerstoff.

7. Verwendung zumindest eines Komplexes nach einem der Ansprüche 1 bis 5 als Katalysator zur Flüssigphasenoxidation von Isoparaffinen zu einem Oxidationsprodukt, das reich an tertiären Alkoholen ist, mit Hilfe eines Gases, das Sauerstoff enthält.

8. Verwendung nach Anspruch 7 in einer Oxidationsreatkion von Isobutan zu tert.Butanol durch Luft.

9. Verwendung nach einem der Ansprüche 7 oder 8, wobei die Temperatur zwischen etwa 40 und 180 °C und der Druck zwischen etwa 1 und 10 MPa liegen.

10. Verwendung nach einem der Ansprüche 7 bis 9, wobei man 0,1 bis 1 Gew.-%, bezogen auf eingesetztes Isoparaffin, an entsprechendem tert.Alkylhydroperoxid oder -peroxid als Reaktionsinitiator verwendet.

11. Verwendung nach einem der Ansprüche 6 bis 10, wobei man eine katalytische Menge des Komplexes von 1 bis 5000 Gew.-ppm, bezogen auf zu oxidierendes Substrat einsetzt.

12. Verwendung als Reagens von zumindest einem Komplex gemäß einem der Ansprüche 1 bis 5 zur Oxidation von linearen oder verzweigten Alkanen, linearen oder cyclischen Olefinen oder alkyl- oder cycloalkylaromatischen Kohlenwasserstoffen zu Alkoholen, Ketonen oder einem Gemisch von Alkoholen und Ketonen.

## Claims

1. Metal peroxide complexes of the general formula LnMXpY [1] wherein L is a ligand of formula (A),

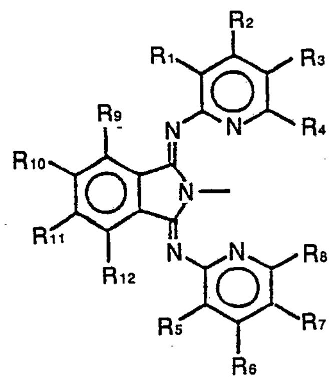

(A)

in which n = 1 or 2, p = 0 or 1 and n + p = 2, both ligands L eing either identical or different when n = 2,

$R_1$ to $R_{12}$, identical or different, are each selected from the group consisting of hydrogen atom, an alkyl group, an aral group, an aralkyl group, an alkoxy group, an alkoxy-alkyl group, a halogen atom or a nitro group,

two adjacent groups $R_1$ to $R_{12}$ optionally forming an aromatic ring together with the carbon atoms to which they are linked,

X represents an anionic group selected from a hydroxide, mu-oxide or mu-peroxide anion or a carboxylate anion selected itself from acetate, propionate, benzoate, pivalate, stearate, 2-ethyl-hexanoate, octoate, naphthenate, nitrobenzoate or trifluoroacetate,

M represents a metal selected from groups VIII, $I_B$ and $VII_B$ and

Y is a peroxo, mu-peroxo group or a group of formula OOR wherein R is an alkyl or aralkyl group.

2. Complexes according to claim 1, wherein M is a metal from group VIII, Y is a group of formula OOR in which R is a tertiary alkyl or tertiary aralkyl group.

3. Complexes according to claim 1 or 2, wherein each of $R_1$ to $R_{12}$, in ligand L, represents a hydrogen atom.

4. Complexes according to one of claims 1 to 3, wherein $R_2$ and $R_6$ simultaneously represent a methyl or tert-butyl group, $R_{11}$ is a methyl or tert-butyl group, $R_1$, $R_3$, $R_4$, $R_5$, $R_7$–$R_{10}$ and $R_{12}$ each represent a hydrogen atom.

5. Complexes according to one of claims 1 to 4, wherein X is a carboxylate group, M is cobalt and $n=p=1$.

6. The use of at least one complex as defined in one of claims 1 to 5 as catalyst for oxidizing olefins, linear or branched alkanes, alkyl or cycloalkyl-aromatic hydrocarbons to alcohols, ketones or a mixture of alcohols and ketones, by means of organic hydroperoxides, in the presence or absence of oxygen.

7. The use of at least one complex as defined in one of claims 1 to 5 as catalyst for oxidizing iso-paraffins in liquid phase to oxidates of high tertiary alcohols content, by means of oxygen-containing gas.

8. The use of a complex according to claim 7, for oxidizing isobutane to tert-butanol, by means of air.

9. The use of a complex according to one of claims 7 or 8, wherein the temperature is about 40–180 °C and the pressure about 1–10 MpA.

10. The use of a complex according to one of claims 7 to 9 wherein 0.1–1% by weight, with respect to the isoparaffin charge, of corresponding tert-alkyl hydroperoxide or peroxide is used as reaction initiator.

11. The use according to one of claims 6 to 10 wherein the catalytic complex is used in an amount of 1 to 5,000 ppm by weight with respect to the substrate to oxidize.

12. The use of at least one complex as defined in one of claims 1 to 5, as reactant for oxidizing linear or branched alkanes, linear or cyclic olefins, alkyl or cycloalkyl-aromatic hydrocarbones, to alcohols, ketones or a mixture of alcohols and ketones.